# EUROPEAN PATENT APPLICATION

(11) **EP 3 701 878 A1**
(43) Date of publication of application: **02.09.2020**
(21) Application number: 18871172.5
(22) Date of filing: 12.10.2018
(51) Int. Cl.: A61B 10/00

(54) **DEMENTIA DETERMINATION SYSTEM**

(30) Priority: 23.10.2017 JP 2017204388; 15.06.2018 JP 2018114516
(71) Applicant: Panasonic Intellectual Property Management Co., Ltd., Osaka-shi, Osaka 540-6207 (JP)
(72) Inventor: NAKAJIMA, Hirobumi, Osaka 540-6207 (JP); MATSUMURA, Yoshihiro, Osaka 540-6207 (JP); NISHIYAMA, Takashi, Osaka 540-6207 (JP); ABE, Kengo, Osaka 540-6207 (JP); SUMI, Sadayuki, Osaka 540-6207 (JP); SASABE, Kohji, Osaka 540-6207 (JP); TAKAHASHI, Atsushi, Osaka 540-6207 (JP); KOIZUMI, Kyohei, Osaka 540-6207 (JP)
(74) Representative: Appelt, Christian W.
(86) International application number: PCT/JP2018/038075
(87) International publication number: WO 2019/082691

(57) **Abstract**

A dementia determination system (100) includes: an acquisition unit (111) configured to acquire a long press ratio which is a ratio of a number of long presses in which a button of an operating device (140) is pressed for a time longer than a reference time, to a number of presses of the button of the operating device (140) used by a user to operate an electric device (130); a determination unit (112) configured to determine a dementia level of the user based on the long press ratio acquired by the acquisition unit (111); and an output unit (113) configured to output dementia information indicating the dementia level determined by the determination unit (112), wherein the determination unit (112) is configured to determine that the dementia level is more severe as the long press ratio is lower.

## Description

### TECHNICAL FIELD

The present invention relates to a dementia determination system and the like for determining a dementia level.

### BACKGROUND ART

Patent Literature 1 (PTL 1) discloses acquiring an operation history of a household electric appliance, determining whether or not an operator has a possibility of dementia based on the operation history, and outputting information indicating that the operator has a possibility of dementia when the operator is determined to have a possibility of dementia. In addition, PTL 1 discloses determining whether or not the operator has a possibility of dementia based on the number of erroneous operations.

### Citation List

### Patent Literature

PTL 1: Japanese Unexamined Patent Application Publication No. 2017-104289
PTL 2: Japanese Unexamined Patent Application Publication No. 2004-135824

### SUMMARY OF THE INVENTION

### TECHNICAL PROBLEMS

However, for example, in the case of mild dementia or the like, the erroneous operations described in PTL 1 may not occur so often. For this reason, mild dementia or the like may not be properly detected. In addition, it may be difficult to determine whether or not an operation performed by the operator is an erroneous operation. Therefore, it is not easy to appropriately determine a dementia level related to whether or not the operator has dementia and the like.

Therefore, an object of the present invention is to provide a dementia determination system or the like that can appropriately determine a dementia level.

### SOLUTIONS TO PROBLEMS

The dementia determination system according to one aspect of the present invention includes: an acquisition unit configured to acquire a long press ratio which is a ratio of a number of long presses in which a button of an operating device is pressed for a time longer than a reference time, to a number of presses of the button of the operating device used by a user to operate an electric device; a determination unit configured to determine a dementia level of the user based on the long press ratio acquired by the acquisition unit; and an output unit configured to output dementia information indicating the dementia level determined by the determination unit, wherein the determination unit is configured to determine that the dementia level is more severe as the long press ratio is lower.

The program according to one aspect of the present invention is a program for causing a computer to execute a dementia determination process, the dementia determination process including: an acquisition step configured to acquire a long press ratio which is a ratio of a number of long presses in which a button of an operating device is pressed for a time longer than a reference time, to a number of presses of the button of the operating device used by a user to operate an electric device; a determination step configured to determine a dementia level of the user based on the long press ratio acquired by the acquisition step; and an output step configured to output dementia information indicating the dementia level determined by the determination step, wherein the determination step is configured to determine that the dementia level is more severe as the long press ratio is lower.

### ADVANTAGEOUS EFFECT OF INVENTION

The dementia determination system or the like according to one aspect of the present invention can appropriately determine a dementia level.

### BRIEF DESCRIPTION OF DRAWINGS

FIG. 1 is a conceptual diagram showing a configuration of a dementia determination system according to Embodiment 1.
FIG. 2 is a block diagram showing a configuration of a dementia determination device according to Embodiment 1.
FIG. 3 is a time chart showing a timing at which the operating device according to Embodiment 1 transmits a signal.
FIG. 4 is a data configuration diagram showing a signal detection result according to Embodiment 1.
FIG. 5 is a graph showing a relationship between a healthy level and the number of presses.
FIG. 6 is a graph showing a relationship between a healthy level and a long press ratio.
FIG. 7 is a flowchart showing an operation of the dementia determination device according to Embodiment 1.
FIG. 8 is a conceptual diagram showing a configuration of a dementia determination system in a variation.
FIG. 9 is a screen transition diagram showing transition of information displayed on a screen of an electric device in the variation.
FIG. 10 is a screen configuration diagram showing a display area of information displayed on the screen of the electric device in the variation.
FIG. 11 is a flowchart showing the operation of the dementia determination device according to the variation.
Fig. 12 is a diagram showing a relative frequency distribution of durations of pressing of a healthy person, an MCI patient, and an AD patient.
FIG. 13 is a diagram showing a correlation between a feature value and a score of the forgetfulness consultation program (MSP) for a plurality of users.
FIG. 14 is a flowchart of the operation of the dementia determination device according to Embodiment 2.

### DESCRIPTION OF EXEMPLARY EMBODIMENTS

Hereinafter, embodiments of the present invention will be described in details with reference to the drawings. Note that each of the embodiments described below shows a comprehensive or specific example. Numerical values, shapes, materials, components, arrangement positions and connecting forms of components, operation orders, and the like shown in the following embodiments are merely examples, and do not limit the present invention. In addition, among the components in the following embodiments, components not described in the independent claims indicating the highest concept are described as arbitrary components.

In addition, each of a plurality of drawings used in the following description is a schematic diagram, and does not necessarily show strictly accurate values and the like.

### EMBODIMENT 1

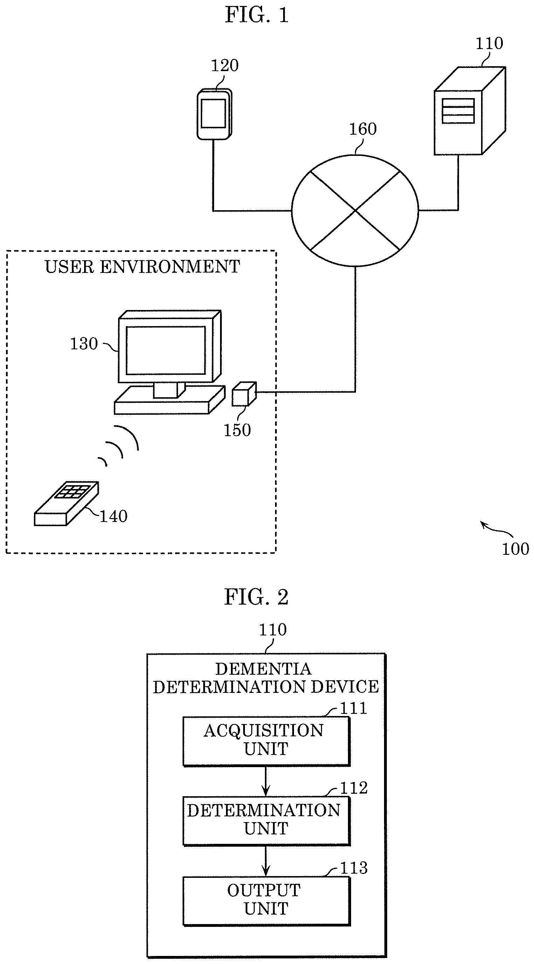

FIG. 1 is a conceptual diagram showing the configuration of the dementia determination system according to Embodiment 1. Dementia determination system 100 shown in FIG. 1 is a system for determining a dementia level, and includes dementia determination device 110, terminal device 120, electric device 130, operating device 140, sensor device 150, and the like.

Here, the dementia level corresponds to the degree of dementia. The dementia level may be the degree of suspicion of dementia or the possibility of dementia. In addition, a level corresponding to a range of a healthy person, a level corresponding to a range of mild cognitive impairment (MCI: Mild Cognitive Impairment) which is milder than dementia, and the like may be set as dementia levels, respectively.

In addition, the dementia level may be set in two stages: a normal level corresponding to a range of a healthy person and an abnormal level corresponding to a range of mild cognitive impairment and dementia. Then, the abnormal level may be further subdivided into a plurality of sub-levels, such as mild cognitive impairment, mild dementia, moderate dementia, and severe dementia.

Dementia determination device 110 is a device for determining a dementia level. That is, the dementia determination in dementia determination system 100 is substantially performed in dementia determination device 110. For example, dementia determination device 110 is a computer that performs information processing. Dementia determination device 110 may be a single device or may include a plurality of auxiliary devices. In addition, dementia determination device 110 may be installed in a user environment, or may be installed in a position different from the user environment.

In the example of FIG. 1, dementia determination device 110 is installed at a position different from the user environment. Then, dementia determination device 110 is connected via a communication network 160 to sensor device 150 installed in a user environment. Then, dementia determination device 110 acquires a detection result from sensor device 150.

In addition, dementia determination device 110 is connected to terminal device 120 via communication network 160. For example, dementia determination device 110 outputs dementia information by transmitting dementia information indicating a dementia level to terminal device 120.

Terminal device 120 is a device that receives the dementia information transmitted from dementia determination device 110. For example, terminal device 120 is a computer that communicates information, and is specifically a tablet terminal, a smart phone, a mobile phone, a personal computer, or the like. Terminal device 120 may be fixedly installed or may be portable.

In addition, terminal device 120 may be installed in a user environment, or may be installed in a position different from the user environment. For example, terminal device 120 is used by a caregiver or the like of a user in a user environment. Terminal device 120 may be used by a user in a user environment.

Electric device 130 is a device operated by a user using operating device 140. For example, electric device 130 is a household electric appliance, and is basically a television receiver. A television receiver is also simply called a television. Electric device 130 may be an audio device, an air conditioner, a lighting device, a refrigerator, a rice cooker, a multifunctional telephone, or the like. In addition, electric device 130 may be a device having a screen. In the example of FIG. 1, electric device 130 is a television. In addition, electric device 130 is basically installed in a user environment.

Operating device 140 is a device for operating electric device 130. For example, operating device 140 is a remote controller for electric device 130 and includes buttons for operating electric device 130. Operating device 140 may be a smart phone or the like for operating electric device 130. Then, buttons for operating electric device 130 may be displayed on the screen included in operating device 140 by a GUI. In addition, operating device 140 may be an operation panel integrated with electric device 130.

Specifically, in the example of FIG. 1, operating device 140 is a remote controller for electric device 130 which is a television. Then, operating device 140 transmits a signal using infrared rays. Thereby, electric device 130 which is a television is operated. In addition, operating device 140 is basically used in a user environment.

Sensor device 150 is a device that detects a signal transmitted from operating device 140. For example, sensor device 150 is an infrared sensor. Sensor device 150 is basically installed near electric device 130. Thereby, sensor device 150 can appropriately detect a signal transmitted from operating device 140. In addition, for example, sensor device 150 transmits a detection result to dementia determination device 110. Sensor device 150 may transmit a detection result to dementia determination device 110 each time a signal is detected.

Alternatively, sensor device 150 may record and store the detection result in an internal memory. Then, when receiving the transmission request of the detection result from dementia determination device 110, sensor device 150 may transmit the detection result recorded and stored in the internal memory.

Communication network 160 is a network for dementia determination device 110, terminal device 120, sensor device 150, and the like to communicate. For example, communication network 160 is the Internet. Communication network 160 may be a LAN (local area network) or the like.

Note that dementia determination device 110 may be dementia determination system 100. In other words, dementia determination system 100 may include the components of dementia determination device 110, and may not include the other components. Then, the other components may be included in other systems. In addition, the communication between the components may be wired communication or wireless communication. Then, communication network 160 may be a wired network or a wireless network.

In addition, although FIG. 1 shows one user environment, a plurality of user environments corresponding to a plurality of users may exist. Then, components corresponding to electric device 130, operating device 140, and sensor device 150 may be included in each user environment. Then, dementia determination device 110 may determine the dementia level of each user.

In addition, for example, one user environment may correspond to one room for the elderly in an apartment complex for the elderly. Then, terminal device 120 may be installed in the management room of the apartment complex for the elderly. Dementia determination device 110 may be installed in the management room of the apartment complex for the elderly, or may be installed outside because it determines a dementia level of each user in a plurality of apartment complexes for the elderly.

FIG. 2 is a block diagram showing the configuration of dementia determination device 110 shown in FIG. 1. Dementia determination device 110 includes acquisition unit 111, determination unit 112, and output unit 113.

Acquisition unit 111 is a processing unit that acquires information, and specifically acquires information for determining a dementia level. Acquisition unit 111 may be a general-purpose or dedicated electric circuit.

For example, acquisition unit 111 may acquire information by receiving information through wired or wireless communication. In this case, acquisition unit 111 may include a terminal or an antenna for communication. Specifically, acquisition unit 111 may acquire the detection result of the signal transmitted from operating device 140 as information by receiving the detection result of the signal transmitted from operating device 140 as information from sensor device 150.

In addition, for example, acquisition unit 111 may receive a detection result of a signal transmitted from operating device 140 from sensor device 150 and acquire information for determining a dementia level from the detection result.

In addition, for example, acquisition unit 111 may acquire information via an input interface such as a mouse, a keyboard, or a touch panel. In addition, acquisition unit 111 may include such an input interface. In addition, acquisition unit 111 may acquire information from a memory such as a recording medium. In addition, acquisition unit 111 may include a connection interface for connecting to a recording medium or the like. In addition, acquisition unit 111 may perform information processing on the acquired information and acquire a result of the information processing as new information.

More specifically, acquisition unit 111 acquires a long press ratio with respect to operating device 140 as information for determining a dementia level. The long press ratio is a ratio of the number of long presses to the number of presses. The number of presses is the number of times a button of operating device 140 is pressed. The number of long presses is the number of times the button of operating device 140 is continuously pressed for a time longer than a predetermined reference time. The reference time may be 3 seconds, 4 seconds, or another time. The reference time may be a fixed time or a dynamically determined time.

In addition, for example, acquisition unit 111 may acquire the number of presses or the number of long presses. Specifically, acquisition unit 111 may acquire the number of presses and the number of long presses from the detection result obtained from sensor device 150. Then, acquisition unit 111 may obtain the long press ratio by calculating the long press ratio from the number of presses and the number of long presses.

Determination unit 112 is a processing unit that determines a dementia level, and specifically determines a dementia level according to the information acquired by acquisition unit 111. Determination unit 112 may be a general-purpose or dedicated electric circuit.

Specifically, determination unit 112 determines the dementia level based on the long press ratio acquired by acquisition unit 111. In particular, determination unit 112 determines that the dementia level is relatively more severe as the long press ratio is lower. For example, when the long press ratio is the first ratio, determination unit 112 determines the dementia level to be the first level, and when the long press ratio is the second ratio lower than the first ratio, determination unit 112 determines the dementia level to be the second level that is more severe than the first level.

In addition, determination unit 112 does not always need to determine that the dementia level is more severe as the long press ratio is lower. According to other conditions and the like, determination unit 112 does not need to exceptionally determine that the dementia level is more severe as the long press ratio is lower. In addition, for example, when the long press ratio is within a predetermined range, determination unit 112 may determine that the dementia level is the same level.

In addition, for example, when the long press ratio is greater than or equal to a predetermined threshold, determination unit 112 may determine the dementia level to be a normal level corresponding to the range of a healthy person. Then, when the long press ratio is lower than the predetermined threshold, determination unit 112 may determine the dementia level to be an abnormal level corresponding to the range of mild cognitive impairment and dementia.

In addition, determination unit 112 may use other parameters for determining the dementia level, without being limited to the long press ratio. That is, determination unit 112 may determine the dementia level based on the long press ratio and other parameters.

The other parameter is, for example, the number of presses acquired by acquisition unit 111. For example, determination unit 112 determines that the dementia level is more severe as the number of presses is smaller. Determination unit 112 may determine that the dementia level is more severe as the long press ratio is lower and the number of presses is smaller, based on the evaluation value obtained by weighting and adding the long press ratio and the number of presses.

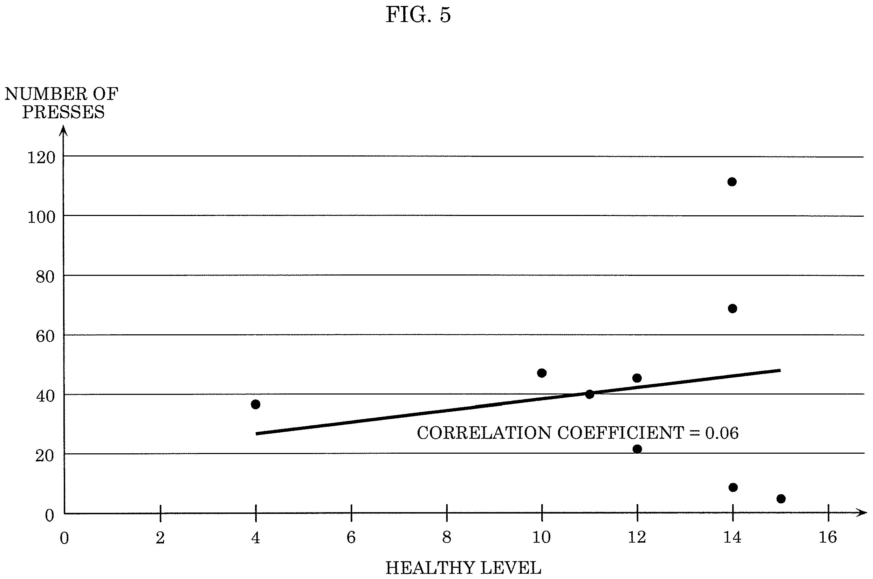

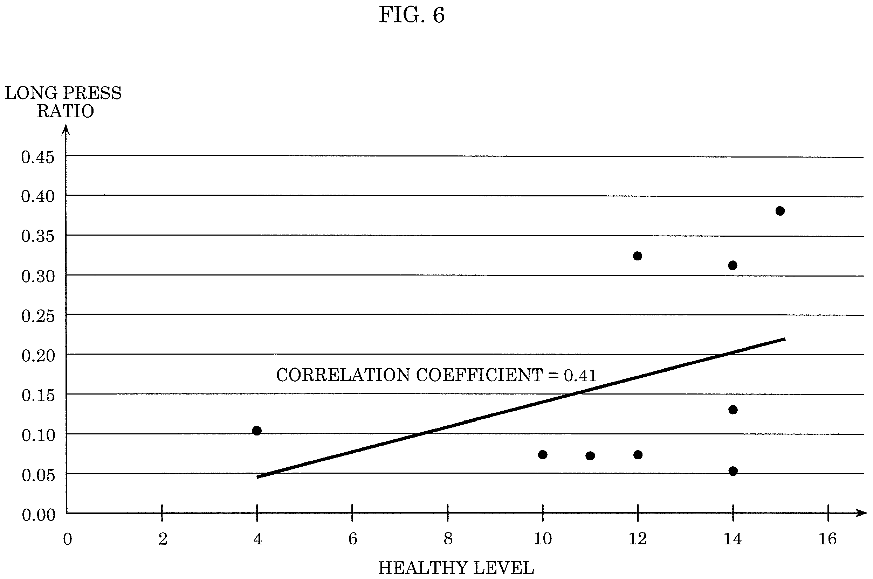

Note that the relationship between the dementia level (healthy level) and the number of presses, and the relationship between the dementia level (healthy level) and the long press ratio will be described later with reference to FIGS. 5 and 6.

Output unit 113 is a processing unit that outputs information, and specifically outputs dementia information indicating the dementia level determined by determination unit 112, and the like. Output unit 113 may be a general-purpose or dedicated electric circuit.

For example, output unit 113 may output information by transmitting information by wire or wireless communication. In this case, output unit 113 may include a terminal or an antenna for communication. Then, output unit 113 may output dementia information by transmitting dementia information to terminal device 120.

In addition, for example, output unit 113 may output information via an output interface such as a display, a speaker, or a touch panel. Specifically, output unit 113 may output the information by displaying the information on a display, or may output the information as audio from a speaker. In addition, output unit 113 may include such an output interface.

In addition, output unit 113 may output information to a memory such as a recording medium. In addition, output unit 113 may include a connection interface for connecting to a recording medium or the like.

In addition, the configuration of the terminal, antenna, input interface, connection interface, or the like in acquisition unit 111 and the configuration of the terminal, antenna, input interface, connection interface, or the like in output unit 113 may be partially or wholly shared.

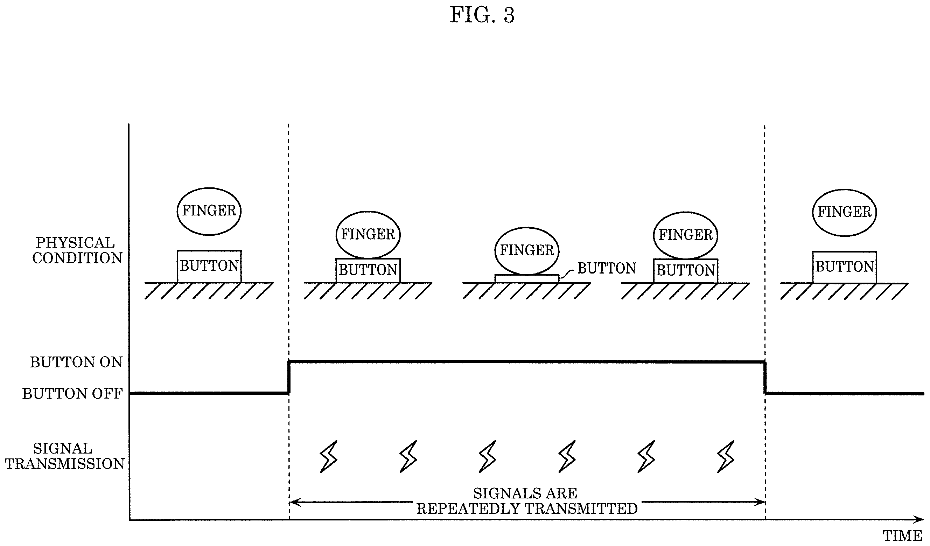

FIG. 3 is a time chart showing the timing at which operating device 140 shown in FIG. 1 transmits a signal.

While the button of operating device 140 is being pressed by the user's finger, the button is in an on state, and the signal is repeatedly transmitted. In particular, the button is in an on state from the time the button starts to be pressed until the time when the button returns. Therefore, the signal is repeatedly transmitted from the time when the button starts to be pressed until the time when the button returns. For example, this signal is transmitted by infrared rays approximately every 200 ms when the button is in an on state. Therefore, if the time period from the time when the button starts to be pressed until the time when the button returns is 1 to 2 seconds, the signal is transmitted 5 to 10 times.

Electric device 130 receives the signal transmitted from operating device 140 and operates according to the received signal. For example, at that time, electric device 130 stops receiving the signal during a few seconds after receiving the signal. Alternatively, electric device 130 discards the signal received during the few seconds. Thereby, electric device 130 suppresses a double operation.

Sensor device 150 also receives a signal transmitted from operating device 140, similarly to electric device 130. That is, sensor device 150 detects a signal transmitted from operating device 140. However, even after detecting a signal, sensor device 150 detects the signal without stopping the detection. Then, sensor device 150 does not discard the signal detected later, but also includes information indicating the signal detected later in the detection result.

Thereby, information indicating a signal transmitted a plurality of times for one press is included in the detection result.

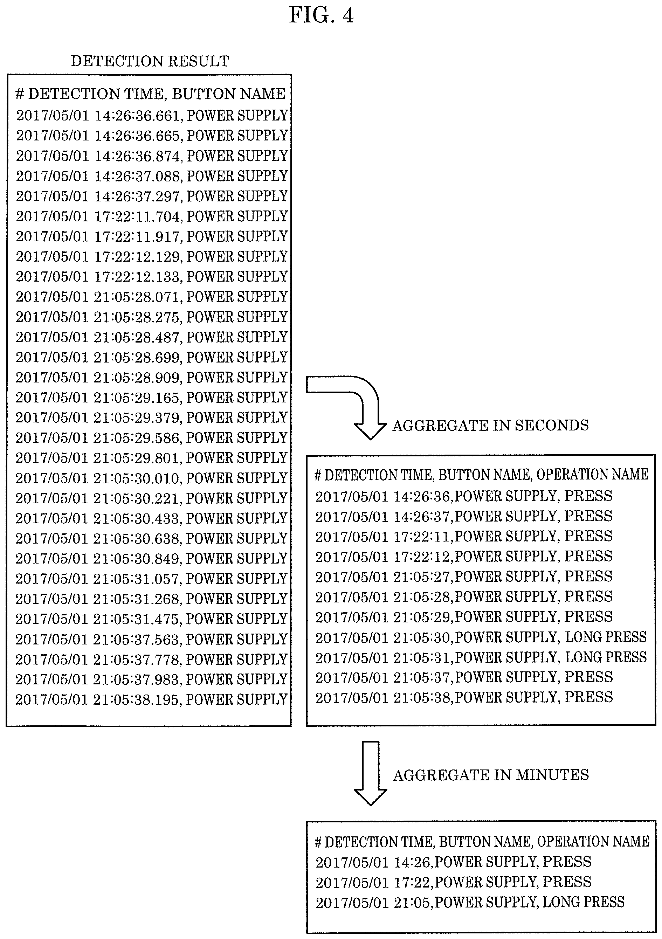

FIG. 4 is a data configuration diagram showing a detection result of a signal for dementia determining device 110 shown in FIG. 2 and the like to determine a dementia level. The detection result shown on the left side of FIG. 4 is an example of the detection result obtained by sensor device 150.

For example, the detection result includes the detection time and the button name. The detection time is a time when sensor device 150 detects a signal, and is a time when sensor device 150 receives a signal transmitted from operating device 140. The button name is a name of the button identified by the detected signal as the pressed button.

Note that in the example of FIG. 4, the detection result of a signal transmitted when the power button is pressed is shown so as not to complicate the description. The detection result obtained by sensor device 150 may include a detection result of a signal transmitted when another button is pressed. In addition, the detection result may be expressed in another format. For example, a button identifier may be used instead of a button name.

Sensor device 150 detects a signal transmitted from operating device 140 in milliseconds. Therefore, the original detection result is a detection result in milliseconds. Then, the detection result in milliseconds is transmitted from sensor device 150 to dementia determination device 110. Acquisition unit 111 of dementia determination device 110 acquires the detection result by receiving the detection result transmitted from sensor device 150.

Then, acquisition unit 111 aggregates the detection results in milliseconds into the detection results in seconds. Although a signal may be detected a plurality of times in one second, it is difficult for the user whose dementia level is determined to press the button a plurality of times in one second. Therefore, it is assumed that a plurality of signal detections in one second correspond to one button press. Therefore, when the signal is detected one or more times in one second, acquisition unit 111 regards that the button is pressed during the one second, and causes the information indicating a press of the button as the operation name to be included in the detection result in seconds.

Furthermore, when the button is continuously pressed for longer than three seconds, acquisition unit 111 regards the button as being long-pressed, and causes the information indicating a long press of the button as the operation name to be included in the detection result in seconds. The three seconds is an example of a time length for identifying a long press, and the time length for identifying a long press may be four seconds or another time length.

Then, acquisition unit 111 aggregates the detection results in seconds into the detection results in minutes. At this time, when there is one or more button presses in one minute, acquisition unit 111 causes the information indicating that there is a press of the button in one minute to be included in the detection result. In addition, when there is a long press of the button in the one minute, acquisition unit 111 causes the information indicating that there is a long press of the button in the one minute to be included in the detection result. This suppresses a single press of the button over a plurality of seconds from being treated as a plurality of presses of the button or a plurality of long presses of the button.

Then, acquisition unit 111 acquires the number of presses of the button and the number of long presses of the button based on the detection result in minutes. Specifically, acquisition unit 111 acquires the total number of presses of the button and long presses of the button included in the detection result in minutes as the number of presses of the button. In addition, acquisition unit 111 acquires the total number of long presses of the button included in the detection result in minutes as the number of long presses of the button.

Then, acquisition unit 111 acquires the ratio of the number of long presses of the button to the number of presses of the button as the long press ratio. In the example of FIG. 4, 1/3 is acquired as the long press ratio.

Note that the format of the detection result, the method of acquiring the number of presses, the method of acquiring the number of long presses, and the method of acquiring the ratio of long presses described above are merely examples, and are not limited to the above examples.

For example, a state in which a signal is detected a plurality of times continuously at a time interval shorter than a predetermined time interval is assumed to be a state in which a button is pressed once. Therefore, a plurality of continuous signal detections may be aggregated into one button press. Then, information indicating the time length from the first detection time in the plurality of detections to the last detection time in the plurality of detections may be added to the aggregated result. Based on such an aggregated result, the number of presses is appropriately acquired. In addition, whether or not each press is a long press is appropriately identified by the additional information.

FIG. 5 is a graph showing the relationship between the healthy level and the number of presses. Specifically, this graph shows the measurement results of the healthy level and the number of presses for nine subjects. The vertical axis indicates the number of presses per day. The horizontal axis indicates the healthy level of the subject.

The healthy level is measured using TDAS (Touch panel type Dementia Assessment Scale) and the like separately from dementia determination system 100, and the healthy level shows more healthy condition as the healthy level is larger. That is, the healthy level of the subject is lower as the dementia of the subject is more severe. Then, the measurement results of nine subjects are plotted on the graph.

In the measurement results, a positive correlation is observed between the healthy level and the number of presses. Therefore, determination unit 112 of dementia determination device 110 may determine that the dementia level is more severe as the number of presses is lower.

However, the correlation coefficient indicating the strength of the relationship between the healthy level and the number of presses is 0.06, and the correlation between the healthy level and the number of presses is weak. In addition, when the subject does not use electric device 130 so often, that is, when the subject does not watch television so often, the number of presses is small. Therefore, even if the number of presses is small, the dementia level may not be severe.

FIG. 6 is a graph showing the relationship between the healthy level and the long press ratio. Specifically, this graph shows the measurement results of the healthy level and the long press ratio for nine subjects. The vertical axis indicates the long press ratio per day. In this example, pressing the button for 4 seconds or longer is a long press. The horizontal axis indicates the healthy level of the subject, as in the example of FIG. 5. Then, similarly to the example of FIG. 5, the measurement results of nine subjects are plotted on the graph.

In the measurement results, a positive correlation is observed between the healthy level and the long press ratio. In addition, the correlation coefficient indicating the strength of the relationship between the healthy level and the long press ratio is 0.41, and the correlation between the healthy level and the long press ratio is larger than the correlation between the healthy level and the number of presses. In addition, it is assumed that the frequency of use of electric device 130 of the subject, specifically, the frequency of viewing television of the subject, has a small effect on the long press ratio.

Then, determination unit 112 of dementia determination device 110 determines that the dementia level is more severe as the long press ratio is lower. Thereby, determination unit 112 of dementia determination device 110 can appropriately determine the dementia level without being affected by the subject's preference or the like.

Note that it is presumed that the will and motivation will decrease and the operating ability will also decrease due to a decrease in cognitive ability. Then thereby, it is presumed that the long press ratio decreases. In the above example, a button press for 4 seconds or longer is set as a long press, but the present invention is not limited to such a time length, and it is presumed that a long press ratio decreases due to a decrease in cognitive ability.

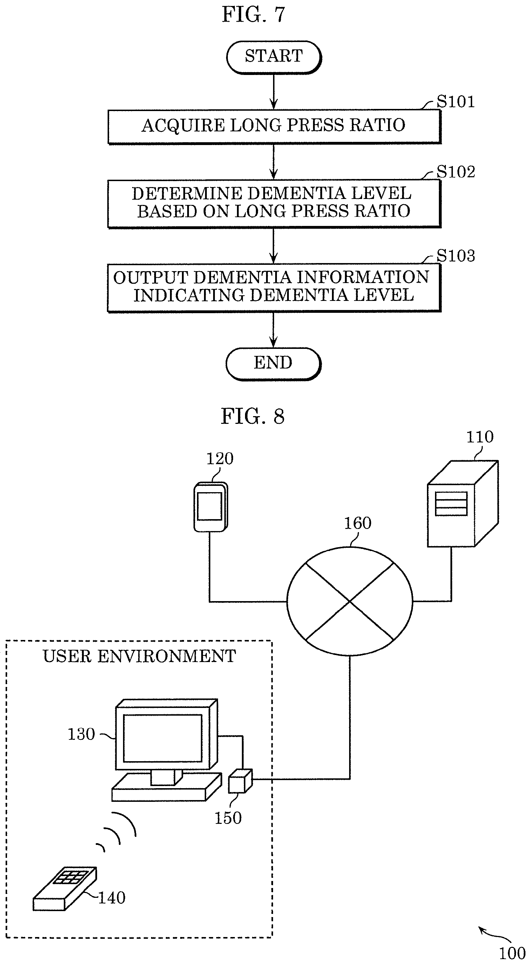

FIG. 7 is a flowchart showing the operation of dementia determination device 110 shown in FIG. 2 and the like. Dementia determination device 110 performs the operation shown in FIG. 7 in determining the dementia level of the user.

First, acquisition unit 111 of dementia determination device 110 acquires a long press ratio (S101). The long press ratio is a ratio of a number of long presses in which a button of operating device 140 is pressed for a time longer than a reference time, to a number of presses of the button of operating device 140 used by a user to operate electric device 130. For example, acquisition unit 111 may acquire a detection result from sensor device 150 that detects a signal transmitted from operating device 140, and may acquire a long press ratio from the detection result.

Next, determination unit 112 of dementia determination device 110 determines the dementia level of the user based on the long press ratio acquired by acquisition unit 111 (S102). At that time, determination unit 112 determines that the dementia level is more severe as the long press ratio is lower. For example, when the long press ratio is equal to or larger than a threshold, determination unit 112 may determine the dementia level to be a normal level corresponding to the range of a healthy person. Then, when the long press ratio is lower than the threshold, the dementia level may be determined to be an abnormal level corresponding to the range of mild cognitive impairment and dementia.

Then, output unit 113 of dementia determination device 110 outputs dementia information indicating the dementia level determined by determination unit 112 (S103). For example, output unit 113 may output dementia information by transmitting the dementia information to terminal device 120.

Thereby, dementia determination device 110 can determine the dementia level according to the daily work performed by the user. In addition, the long press ratio for determining the dementia level is set irrespective of whether the operation is correct or erroneous, and is hardly influenced by user's preference. Then, it is presumed that the long press ratio decreases as the cognitive ability decreases. Therefore, dementia determination device 110 can appropriately determine the dementia level based on the long press ratio.

### (Variation)

FIG. 8 is a conceptual diagram showing a varied configuration of dementia determination system 100 shown in FIG. 1. In this variation, sensor device 150 is connected to electric device 130 and controls the operation of electric device 130 by communicating with electric device 130. That is, sensor device 150 operates as a control device that controls the operation of electric device 130.

For example, output unit 113 of dementia determination device 110 may display the dementia information on the screen of electric device 130 via sensor device 150 by outputting the dementia information. Specifically, output unit 113 of dementia determination device 110 outputs dementia information to sensor device 150. Then, sensor device 150 receives the dementia information from dementia determination device 110, and transmits the dementia information to electric device 130. Then, electric device 130 receives the dementia information from sensor device 150 and displays the dementia information on the screen.

Thereby, dementia determination device 110 can notify the user of electric device 130 of the dementia level.

In addition, for example, by outputting training content for improving the cognitive ability of the user, output unit 113 of dementia determination device 110 may display the training content on the screen of electric device 130 via sensor device 150.

Specifically, similarly to the example of the dementia information, output unit 113 of dementia determination device 110 outputs the training content to sensor device 150 based on the dementia level. Then, sensor device 150 receives the training content from dementia determination device 110, and transmits the training content to electric device 130. Then, electric device 130 receives the training content from sensor device 150 and displays the dementia information on the screen.

Thereby, dementia determination device 110 can prompt the user of electric device 130 to perform training for improving cognitive ability. The training content displayed on the screen of electric device 130 is training content for an operation in which operating device 140 is used. For example, by performing an operation on electric device 130 using operating device 140 according to the training content, training for improving cognitive ability is performed. Since such an operation involves the movement of the user's finger, it is effective in improving cognitive ability.

In addition, training content may be set for each dementia level. Thereby, training for improving the user's cognitive ability is performed according to the training content set for the dementia level of the user. The training content may be recorded in a memory inside or outside dementia determination device 110 in advance.

In addition, when the dementia level of the user is determined to be a level corresponding to mild cognitive impairment or dementia, output unit 113 of dementia determination device 110 may cause the training content to be displayed on the screen of electric device 130. That is, when the determined dementia level is more severe than the predetermined reference level, output unit 113 of dementia determination device 110 may cause the training content to be displayed on the screen of electric device 130. Thereby, dementia determination device 110 can suppress the training content from being displayed when the training is not necessary.

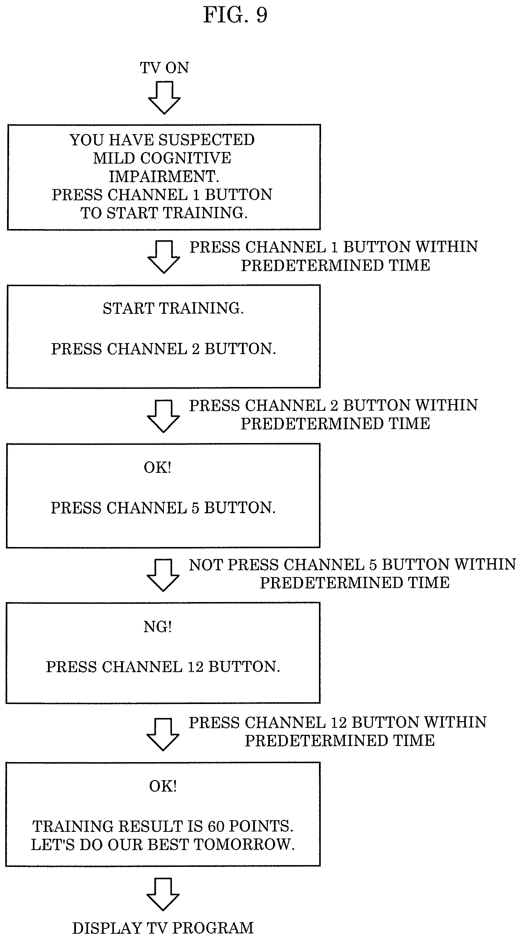

FIG. 9 is a screen transition diagram showing transition of information displayed on the screen of electric device 130 shown in FIG. 8. For example, dementia information and training content are displayed as shown in FIG. 9.

In the example of FIG. 9, when the television, which is electric device 130, is turned on, a notification that there is a suspicion of mild cognitive impairment is displayed on the screen of the television. This notification is displayed when the television is first turned on every day, and may not be displayed after the second time. In addition, this notification corresponds to dementia information indicating a dementia level determined based on a past operation. For example, this notification may indicate a dementia level determined based on the operations during the past day, the past week, the past month or the like.

Then, when the training is started, the user is prompted to press the channel 1 button. Here, the training is started when the channel 1 button is pressed within a predetermined time. That is, the training content is displayed.

For example, when the channel 1 button is pressed within a predetermined time, next, the start of training is notified, and the user is prompted to press the channel 2 button. When the channel 2 button is pressed within a predetermined time, a notification of OK is given, and then the user is prompted to press the channel 5 button. When the channel 5 button has not been pressed within a predetermined time, a notification of NG is given, and then the user is prompted to press the channel 12 button. When the channel 12 button is pressed within a predetermined time, the notification of OK is given.

Then, the training result is displayed by the score. At that time, the score may be set according to the number of times the operation is appropriately performed according to the instruction indicated by the training content. In addition, the long press ratio during training may be reflected in the score. Thereafter, the display of the television program is started as usual.

Note that dementia determination device 110 may reflect the long press ratio during training in the determination of the dementia level. In addition, dementia determination device 110 may newly determine the dementia level based on the long press ratio in the determination target period including the training period, and may display dementia information indicating the newly determined dementia level on the screen after training.

In addition, the predetermined time for pressing the channel button may be set according to the dementia level of the user. In addition, the press of the channel button is repeated three times in the training in the above description, but the number of repetitions may be set based on the dementia level of the user.

For example, a plurality of training contents having different predetermined times for button pressing and different numbers of repetitions may be set for a plurality of dementia levels in advance. Then, the training may be performed according to the training content set for the dementia level of the user among the plurality of training content set for the plurality of dementia levels in advance.

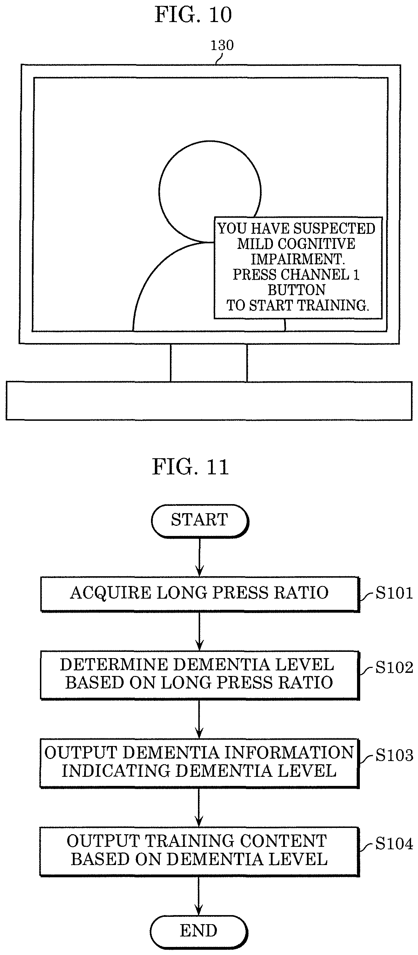

FIG. 10 is a screen configuration diagram showing a display area of information displayed on the screen of electric device 130 shown in FIG. 8. The dementia information and the training content may be displayed in a partial area of the screen instead of the entire screen. In the example of FIG. 10, dementia information and training content are displayed in the lower right area of the screen. Then, the television program is displayed in another area of the screen.

Note that a button that is rarely used may be used as a button used for training and a button used for starting training. Thereby, it is possible to suppress the operation for viewing the television program from being hindered.

FIG. 11 is a flowchart showing the operation of dementia determination system 100 shown in FIG. 8 and the like. Dementia determination device 110 according to the variation performs the operation shown in FIG. 11 in determining the dementia level of the user.

First, acquisition unit 111 of dementia determination device 110 acquires a long press ratio (S101). Next, determination unit 112 of dementia determination device 110 determines the dementia level of the user based on the long press ratio acquired by acquisition unit 111 (S102). Then, output unit 113 of dementia determination device 110 outputs dementia information indicating the dementia level determined by determination unit 112 (S103).

These processes are basically the same as in the example of FIG. 7. In the present variation, output unit 113 of dementia determination device 110 may display the dementia information on the screen of electric device 130 by outputting the dementia information.

In addition, in the present variation, output unit 113 of dementia determination device 110 outputs the training content on the screen of electric device 130 by outputting the training content based on the dementia level determined by determination unit 112 (S104). For example, output unit 113 of dementia determination device 110 may display the training content set for the dementia level on the screen of electric device 130 by outputting the training content set for the dementia level determined by determination unit 112.

Thereby, dementia determination device 110 can notify the user of the determination result of the dementia level via electric device 130 used for the determination of the dementia level. Then thereby, dementia determination device 110 can prompt the user to improve the life rhythm and the like. In addition, dementia determination device 110 can prompt the user to improve cognitive ability by displaying the training content on the screen of electric device 130.

In addition, for example, the operation on electric device 130 is performed using operating device 140 according to the training content. Dementia determination device 110 may reflect the operation performed according to the training content in the next determination.

Specifically, in the next determination, acquisition unit 111 of dementia determination device 110 newly acquires the long press ratio in the determination target period including the training period in which the training content is displayed. Then, determination unit 112 of dementia determination device 110 newly determines the dementia level based on the long press ratio newly acquired by acquisition unit 111.

Thereby, dementia determination device 110 can appropriately determine the dementia level improved by the training.

Note that in Embodiment 1 and the variation, sensor device 150 may be incorporated in electric device 130. Then, electric device 130 may have a role of sensor device 150. Furthermore, dementia determination device 110 may be incorporated in electric device 130, or a plurality of components included in dementia determination device 110 may be incorporated in electric device 130. Then, electric device 130 may have a role of dementia determination device 110.

Dementia determination system 100 may authenticate each user from among a plurality of users in the user environment and determine the dementia level of the authenticated user from among the plurality of users. For example, a plurality of operating devices may be assigned to a plurality of users, respectively. Then, the user may be authenticated according to the operating device used for the operation among the plurality of operating devices. Alternatively, one operating device may authenticate each user by fingerprint authentication.

In addition, when determining the dementia level based on the long press ratio, dementia determination system 100 may compare the long press ratio in the past state where the user was healthy with the long press ratio in the current state. Then, when the long press ratio in the current state is lower than the long press ratio in the past state, dementia determination system 100 may determine that the dementia level in the current state is more severe than the dementia level in the past state.

That is, the threshold for the long press ratio for determining the dementia level of the user in the current state may be set based on the long press ratio in the past state in which the user was healthy. Alternatively, in order to set such a threshold, instead of the long press ratio in a past state where the user was healthy, an average long press ratio in a healthy state of a plurality of users may be used.

In addition, when determining the dementia level based on the long press ratio, dementia determination system 100 may use other determination criteria in addition to the long press ratio. That is, dementia determination system 100 may determine the dementia level based on the long press ratio and other parameters.

In addition, instead of the long press ratio, an average pressing time, that is, a time length per press of the button may be used. The fact that the dementia level is more severe as the long press rate is lower can be replaced by the fact that the dementia level is more severe as the average pressing time is shorter. Therefore, dementia determination system 100 may determine that the dementia level is more severe as the average pressing time is shorter.

### EMBODIMENT 2

### [Issue in Embodiment 2]

In recent years, the ratio of elderly people to the total population has been increasing. With the advent of such an aging society, it is thought that the problem of dementia will become apparent. The issue of dementia has been actively discussed in mass media such as television programs.

There are various levels of dementia ranging from mild to severe, and if it is known that a person is in a stage of mild cognitive impairment (MCI: Moderate Cognitive Impairment) before dementia occurs, the onset of dementia can be suppressed by training or the like. PTL 2 discloses a diagnostic device for higher brain dysfunction as a technique for determining a dementia level.

### [Configuration]

In Embodiment 2, dementia determination system 100 determines the dementia level based on the operation history of the electric device. Differences between the configuration of such dementia determination system 100 and the configuration of Embodiment 1 will be described.

Acquisition unit 111 acquires the duration of pressing the button of operating device 140 as information for determining the dementia level. The duration of pressing is the length of time during which the button is pressed in one press of the button.

Determination unit 112 determines the dementia level based on the relative frequency distribution of the duration of pressing acquired by acquisition unit 111.

### [Findings that led to inventing a cognitive function determination system that performs the operation of Embodiment 2]

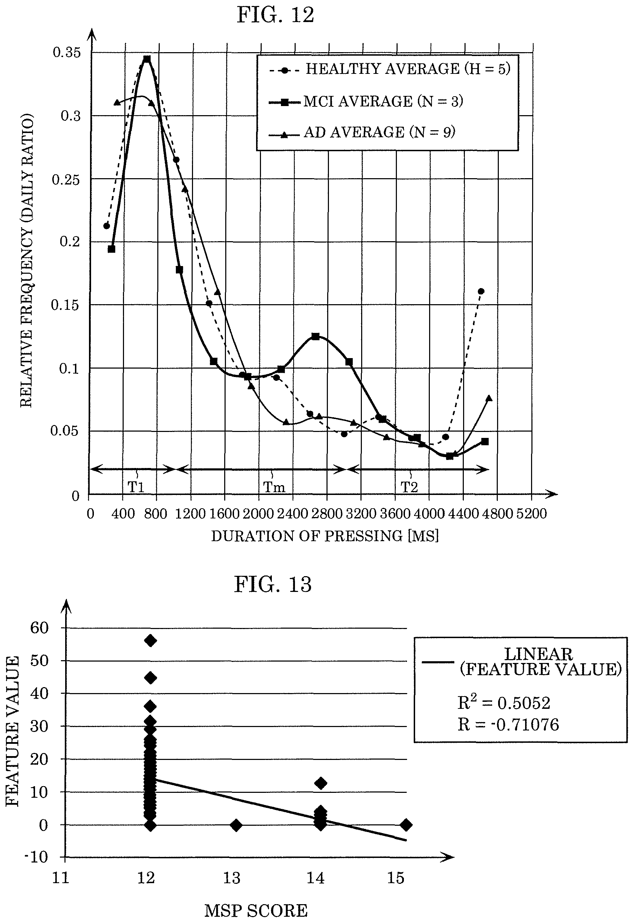

Hereinafter, the findings of the inventors who have invented dementia determination system 100 that performs the operation of Embodiment 2 will be described. The user for which the dementia level is to be determined presses the button of operating device 140 a plurality of times a day. The plurality of presses of the button usually has a different duration of pressing. Then, when the inventors calculated the relative frequency distribution of the duration of pressing for one day, they found that the relative frequency distributions of a healthy person, an MCI patient, and an AD patient had different shapes. FIG. 12 is a diagram showing the relative frequency distributions of the duration of pressing of the healthy person, the MCI patient, and the AD patient. Note that each of the relative frequency distributions of the healthy person, the MCI patient, and the AD patient shown in FIG. 12 is obtained by averaging the relative frequency distributions of a plurality of users. Note that the relative frequency distribution of each user is calculated by averaging the relative frequency distribution of the duration of pressing for one day over a plurality of days (for example, over several months).

As shown in FIG. 12, when the duration of pressing is divided into three continuous sections (specifically, first section T1, intermediate section Tm, and second section T2), only the relative frequency distribution of the MCI patient has a clear peak in intermediate section Tm. That is, in the MCI patient, the case where the duration of pressing is short and the case where the duration of pressing is long decrease, and the case where the duration of pressing has an intermediate value increases. Note that in FIG. 12, intermediate section Tm is, for example, a section of 1000 ms or more and 3000 ms or less, but may be appropriately changed empirically or experimentally.

It is considered that the reason why the peak occurs in intermediate section Tm is that the MCI patient cannot press the button quickly (that is, in a short time) and cannot press the button in a long time due to a decrease in cognitive function.

Therefore, dementia determination system 100 determines the dementia level by using the fact that the relative frequency in the intermediate section Tm is relatively larger than the other two sections (a peak is generated) in the relative frequency distribution of the MCI patient. That is, dementia determination system 100 determines the dementia level by comparing the relative frequency distribution in intermediate section Tm with the relative frequency distribution in the other two sections when the duration of pressing is divided into three continuous sections.

Specifically, when the integrated value of the relative frequency distribution in first section T1 is A, the integrated value of the relative frequency distribution in intermediate section Tm is B, and the integrated value of the relative frequency distribution in second section T2 is C, dementia determination system 100 determines the dementia level of the user based on the feature value represented by (B / A) + (B / C). FIG. 13 is a diagram showing a correlation between a feature value and a score of the forgetfulness consultation program (MSP) for a plurality of users. Note that the forgetfulness consultation program is a screening test program for finding AD patients.

As shown in FIG. 13, the correlation coefficient R between the feature value described above and the MSP score is R = -0.71076 (R² = 0.5052). In addition, the p value in this case is p = 0.00000000009417043, which is extremely lower than the significance level of 0.05. That is, it can be said that the feature value described above and the MSP score have a good correlation. Note that the feature value described above has a larger value as the tendency to be an MCI patient is stronger, whereas it is shown that the cognitive function is decreased as the numerical value of the MSP score is lower, so that both have a negative correlation.

### [Operation]

The operation of determining the dementia level of dementia determination system 100 using the feature value as described above will be described. The method for detecting the signal transmitted from operating device 140 by sensor device 150 is as described in Embodiment 1 with reference to FIG. 3. In addition, the detection result of the signal of sensor device 150 is as described in Embodiment 1 with reference to FIG. 4. Note that it is not essential in Embodiment 2 that the detection results are aggregated. The detection result before being aggregated shown in FIG. 4 indicates the duration of pressing.

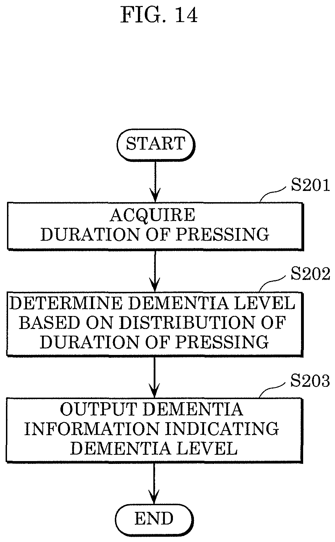

Hereinafter, the operation of dementia determination device 110 using the detection result of sensor device 150 will be described. FIG. 14 is a flowchart of the operation of dementia determination device 110. Dementia determination device 110 performs the operation shown in FIG. 14 in determining the dementia level of the user.

First, acquisition unit 111 of dementia determination device 110 acquires the duration of pressing (S201). The duration of pressing is the duration of pressing the button of operating device 140 used by the user for the operation on electric device 130. For example, acquisition unit 111 acquires a detection result from sensor device 150 that detects a signal transmitted from operating device 140, and acquires the duration of pressing from the detection result.

Next, determination unit 112 of dementia determination device 110 determines the dementia level of the user based on the relative frequency distribution of the duration of pressing acquired by acquisition unit 111 (S202). Determination unit 112 calculates the relative frequency distribution used for the determination by, for example, averaging the relative frequency distribution of the duration of pressing for the user for one day over a plurality of days, and calculates the above-described feature value from the calculated relative frequency distribution. Note that the method of calculating the relative frequency distribution used for the determination is not limited to such a method. For example, the averaging is a process for improving the accuracy of the determination, and is not essential. In addition, it is not essential that the relative frequency distribution is based on the duration of pressing for one day for the user, but may be based on the duration of pressing for a predetermined period.

For example, when the feature value is equal to or larger than a threshold, determination unit 112 determines the dementia level as an abnormal level corresponding to mild cognitive impairment. Note that when the feature value is smaller than the threshold, determination unit 112 may determine that the dementia level is a normal level corresponding to the range of a healthy person or an abnormal level corresponding to the range of dementia. In addition, the abnormal level corresponding to mild cognitive impairment may be further finely divided into a plurality of sub-levels. In this case, determination unit 112 determines that the sub-level has stronger tendency for mild cognitive impairment as the feature value is larger.

Then, output unit 113 of dementia determination device 110 outputs dementia information indicating the dementia level determined by determination unit 112 (S203). For example, output unit 113 may output dementia information by transmitting dementia information to terminal device 120.

As described above, dementia determination device 110 can determine the dementia level of the user based on the operation history of electric device 130. That is, dementia determination device 110 can determine the dementia level according to the daily work performed by the user. In addition, the duration of pressing for determining the dementia level is set irrespective of whether the operation is correct or erroneous, and is hardly influenced by the user's preference. Therefore, dementia determination device 110 can appropriately determine the dementia level based on the duration of pressing.

### [Variation]

In Embodiment 2 described above, determination unit 112 determines the dementia level based on the feature value obtained from the relative frequency distribution, but such a determination method is an example. For example, determination unit 112 may determine the dementia level by obtaining the degree of approximation between the relative frequency distribution of the duration of pressing for the user and a preset reference distribution. For example, when the relative frequency distribution of the standard duration of pressing for the mild cognitive impairment patient is used as the reference distribution, determination unit 112 can determine that the user has stronger tendency for mild cognitive impairment as the relative frequency distribution of the duration of pressing for the user is closer to the reference distribution. Note that when the degree of approximation is obtained, a technique such as machine learning may be used.

In addition, determination unit 112 may determine the dementia level by comparing the peak level (that is, the peak value of the relative frequency) in intermediate section Tm of the relative frequency distribution with a threshold. In this case, determination unit 112 can determine that the user has stronger tendency for mild cognitive impairment as the peak level is higher. In addition, determination unit 112 may determine the dementia level (in this case, whether or not the user has mild cognitive impairment) based on the presence or absence of a peak in intermediate section Tm of the relative frequency distribution.

In addition, determination unit 112 may determine the dementia level of the user based on a temporal change in the relative frequency distribution of the duration of pressing. That is, determination unit 112 may determine the dementia level by comparing the relative frequency distribution of the past duration of pressing for a certain user with the relative frequency distribution of the current duration of pressing for the user. Since such a determination method is based on the change of the same user, it is effective when the determination accuracy cannot be ensured due to individual differences. Specifically, determination unit 112 may determine the dementia level based on a temporal change in the above-described feature value, or may determine the dementia level based on a temporal change in the degree of approximation to the reference distribution. Alternatively, determination unit 112 may determine the dementia level based on a temporal change in the peak level.

In addition, in Embodiment 2 and its variation, the relative frequency distribution is used for determining the dementia level, but a normal frequency distribution may be used instead of the relative frequency distribution. The relative frequency distribution has an advantage that statistical processing such as averaging is easy.

Note that in Embodiment 2 and its variation, sensor device 150 may be incorporated in electric device 130. Then, electric device 130 may have a role of sensor device 150. Furthermore, dementia determination device 110 may be incorporated in electric device 130, or a plurality of components included in dementia determination device 110 may be incorporated in electric device 130. Then, electric device 130 may have a role of dementia determination device 110.

In addition, dementia determination system 100 may authenticate each user from among a plurality of users in the user environment and determine the dementia level of the authenticated user from among the plurality of users. For example, a plurality of operating devices may be assigned to a plurality of users, respectively. Then, the user may be authenticated according to the operating device used for the operation among the plurality of operating devices. Alternatively, one operating device may authenticate each user by face authentication or fingerprint authentication.

### OTHER EMBODIMENTS

As described above, dementia determination system 100 according to one aspect of the present invention has been described according to Embodiments 1 and 2 and the like, but the present invention is not limited to Embodiments 1 and 2 and the like. The present invention includes a form obtained by applying variations that can be conceived by those skilled in the art to Embodiments 1 and 2 and the like, and another form realized by arbitrarily combining a plurality of components in Embodiments 1 and 2 and the like.

For example, a process performed by a specific component may be performed by another component. In addition, the order in which the processes are performed may be changed, or a plurality of processes may be performed in parallel.

In addition, the present invention can be implemented not only as dementia determination system 100 but also as a dementia determination method including steps performed by each component included in dementia determination system 100. For example, those steps are executed by a computer system including a processor, a memory, an input / output circuit, and the like. Then, the present invention can be realized as a program for causing a computer system to execute the steps included in those methods. Note that a computer system may be simply referred to as a computer.

In addition, the present invention can be realized as a non-transitory computer-readable recording medium on which the above-described program is recorded. The recording medium may be an optical disk such as a CD-ROM, a magnetic disk such as a hard disk drive, a magneto-optical disk (MO), a semiconductor memory such as a flash memory, or other non-transitory computer-readable recording medium. In addition, the program may be recorded in a recording medium in advance, or may be recorded in the recording medium by being supplied to the recording medium via a communication network.

For example, when the present invention is implemented by a program, each step is executed by executing the program using hardware resources such as a processor, a memory, and an input / output circuit of a computer system. That is, each step is executed by the processor acquiring data from a memory or an input / output circuit or the like and performing an operation, or outputting the operation result to the memory or the input / output circuit or the like. Any type of processor can be used as the processor for executing the program.

In addition, each of the plurality of components included in dementia determination system 100 and the like may be realized as a dedicated or general-purpose circuit. These components may be realized as one circuit or as a plurality of circuits.

In addition, the plurality of components included in dementia determination system 100 and the like may be realized as an LSI (Large Scale Integration) that is an integrated circuit (IC). These components may be individually integrated into one chip, or may be integrated into one chip so as to include some or all of the components. These components may be provided on one or more chips of one device, or may be provided on a plurality of chips of a plurality of devices.

In addition, the LSI may be called a system LSI, a super LSI, or an ultra LSI depending on the degree of integration. In addition, the integrated circuit may be realized by a dedicated circuit or a general-purpose processor. A programmable FPGA (Field Programmable Gate Array) or a reconfigurable processor in which connection and setting of internal circuit cells can be reconfigured may be used.

Furthermore, if an integrated circuit technology that replaces the LSI appears due to the advancement of the semiconductor technology or another derivative technology, naturally, the integrated circuit of a plurality of components included in dementia determination system 100 may be integrated using the technology.

Finally, a plurality of aspects of dementia determination system 100 and the like will be described as examples. These aspects may be appropriately combined. In addition, any of the features and the like described in Embodiments 1, 2 and the like may be added.

### (Aspect 1)

Dementia determination system 100 according to one aspect of the present invention includes acquisition unit 111, determination unit 112, and output unit 113.

Acquisition unit 111 acquires a long press ratio which is a ratio of a number of long presses in which a button of operating device 140 is pressed for a time longer than a reference time, to a number of presses of the button of operating device 140 used by a user to operate electric device 130. Determination unit 112 determines a dementia level of the user based on the long press ratio acquired by acquisition unit 111. At that time, determination unit 112 determines that the dementia level is more severe as the long press ratio is lower. Output unit 113 outputs dementia information indicating the dementia level determined by determination unit 112.

Thereby, dementia determination system 100 can determine the dementia level according to the daily work performed by the user. In addition, the long press ratio for determining the dementia level is set irrespective of whether the operation is correct or erroneous, and is hardly influenced by user's preference. Then, it is presumed that the long press ratio decreases as the cognitive ability decreases. Therefore, dementia determination system 100 can appropriately determine the dementia level based on the long press ratio.

### (Aspect 2)

For example, when the long press ratio is greater than or equal to the threshold, determination unit 112 may determine that the dementia level is a normal level corresponding to a range of a healthy person. Then, when the long press ratio is lower than the threshold, determination unit 112 may determine that the dementia level is an abnormal level corresponding to a range of mild cognitive impairment and dementia. Thereby, dementia determination system 100 can appropriately determine whether the dementia level of the user is a normal level or an abnormal level.

### (Aspect 3)

For example, output unit 113 may cause the dementia information to be displayed on a screen of electric device 130 by outputting the dementia information. Thereby, dementia determination system 100 can notify the user of the dementia level of the user. Therefore, dementia determination system 100 can prompt the user to improve the life rhythm.

### (Aspect 4)

For example, by outputting training content for an operation in which operating device 140 is used based on the dementia level determined by determination unit 112, output unit 113 may cause the training content to be displayed on a screen of electric device 113. Thereby, dementia determination system 100 can prompt the user for training based on the dementia level determined.

### (Aspect 5)

For example, when the dementia level determined by determination unit 112 is more severe than a reference level, output unit 113 may cause the training content to be displayed on the screen by outputting the training content. Thereby, dementia determination system 100 can prompt the user for training when the user is determined to have mild cognitive impairment or dementia and the like.

### (Aspect 6)

For example, output unit 113 may cause the training content to be displayed on the screen of electric device 130 by outputting the training content set for the dementia level determined by determination unit 112. Thereby, dementia determination system 100 can prompt the user for training suitable for the dementia level determined.

### (Aspect 7)

For example, acquisition unit 111 may newly acquire the long press ratio in the determination target period including a training period in which the training content is displayed. Determination unit 112 may newly determine the dementia level based on the long press ratio newly acquired by acquisition unit 111. Thereby, dementia determination system 100 can collect more information and determine the dementia level. In addition, dementia determination system 100 can determine a dementia level improved by training.

### (Aspect 8)

The program according to one aspect of the present invention is a program for causing a computer to execute a dementia determination process including acquisition step (S101), determination step (S102), and output step (S103).

In acquisition step (S101), a long press ratio is acquired which is a ratio of a number of long presses in which a button of operating device 140 is pressed for a time longer than a reference time, to a number of presses of the button of operating device 140 used by a user to operate electric device 130.

In determination step (S102), a dementia level of the user based on the long press ratio acquired by acquisition step (101) is determined. In addition, it is determined in determination step (S102) that the dementia level is more severe as the long press ratio is lower. In output step (S103), dementia information indicating the dementia level determined by determination step (S102) is output.

Thereby, the program can cause the computer to execute an appropriate determination of the dementia level.

### (Aspect 9)

Dementia determination system 100 according to one aspect of the present invention includes acquisition unit 111, determination unit 112, and output unit 113.

Acquisition unit 111 acquires a duration of pressing a button of operating device 140 used by a user to operate electric device 130. Determination unit 112 determines a dementia level of the user based on a distribution of the duration of pressing acquired. Output unit 113 outputs dementia information indicating the dementia level determined.

Thereby, dementia determination system 100 can determine the dementia level of the user based on the operation history of electric device 130. That is, dementia determination system 100 can determine the dementia level according to the daily work performed by the user. In addition, the duration of pressing for determining the dementia level is set irrespective of whether the operation is correct or erroneous, and is hardly influenced by the user's preference. Therefore, dementia determination system 100 can appropriately determine the dementia level based on the duration of pressing.

### (Aspect 10)

For example, determination unit 112 may determine the dementia level of the user by comparing the distribution in an intermediate section Tm when the duration of pressing is divided into three continuous sections and the distribution in other two sections of the three continuous sections. Thereby, dementia determination system 100 can determine the dementia level of the user based on the comparing the distribution in intermediate section Tm and the distribution in the other two sections.

### (Aspect 11)

For example, when A is an integral value of the distribution in a first section immediately before intermediate section Tm, B is an integral value of the distribution in the intermediate section, and C is an integral value of the distribution in a second section immediately after the intermediate section, determination unit 112 may be determine the dementia level of the user based on a feature value represented by (B / A) + (B / C). Thereby, dementia determination system 100 can determine the dementia level of the user based on the feature value obtained from the distribution.

### (Aspect 12)

For example, when the feature value is greater than or equal to a threshold value, determination unit 112 determines the dementia level to be an abnormal level corresponding to mild cognitive impairment. Thereby, dementia determination system 100 can appropriately determine whether or not the dementia level of the user is an abnormal level corresponding to mild cognitive impairment.

### (Aspect 13)

For example, intermediate section Tm may be a section in which the duration of pressing is 1000 ms or more and 3000 ms or less. Thereby, dementia determination system 100 can determine the dementia level of the user based on a comparison between the distribution in intermediate section Tm having the duration of pressing of 1000 ms or more and 3000 ms or less and the distribution in the other two sections.

### (Aspect 14)

For example, determination unit 112 may determine the dementia level of the user based on a temporal change in the distribution. Thereby, dementia determination system 100 can determine the dementia level of the user based on the temporal change in the distribution.

### (Aspect 15)

For example, the distribution is calculated by averaging a relative frequency distribution of the duration of pressing for one day over a plurality of days. Thereby, dementia determination system 100 can determine the dementia level with high accuracy.

### (Aspect 16)

The program according to one aspect of the present invention is a program for causing a computer to execute a dementia determination process including acquisition step (S201), determination step (S202), and output step (S203).

In acquisition step (S201), a duration of pressing a button of operating device 140 used by a user to operate the electrical device 130 is acquired. In determination step (S202), a dementia level of the user based on a distribution of the duration of pressing acquired is determined. In output step (S203), dementia information indicating the dementia level determined is output.

Thereby, the program can cause the computer to determine the dementia level based on the operation history of electric device 130.

### REFERENCE MARKS IN THE DRAWINGS

- 100: dementia determination system
- 111: acquisition unit
- 112: determination unit
- 113: output unit
- 130: electric device
- 140: operating device

## Claims

1. A dementia determination system, comprising:
an acquisition unit configured to acquire a long press ratio which is a ratio of a number of long presses in which a button of an operating device is pressed for a time longer than a reference time, to a number of presses of the button of the operating device used by a user to operate an electric device;
a determination unit configured to determine a dementia level of the user based on the long press ratio acquired by the acquisition unit; and
an output unit configured to output dementia information indicating the dementia level determined by the determination unit, wherein
the determination unit is configured to determine that the dementia level is more severe as the long press ratio is lower.

2. The dementia determination system according to claim 1, wherein
when the long press ratio is greater than or equal to a threshold, the determination unit is configured to determine that the dementia level is a normal level corresponding to a range of a healthy person, and
when the long press ratio is lower than the threshold, the determination unit is configured to determine that the dementia level is an abnormal level corresponding to a range of mild cognitive impairment and dementia.

3. The dementia determination system according to claim 1 or 2, wherein
the output unit is configured to cause the dementia information to be displayed on a screen of the electric device by outputting the dementia information.

4. The dementia determination system according to any one of claims 1 to 3, wherein
by outputting training content for an operation in which the operating device is used based on the dementia level determined by the determination unit, the output unit is configured to cause the training content to be displayed on a screen of the electric device.

5. The dementia determination system according to claim 4, wherein
when the dementia level determined by the determination unit is more severe than a reference level, the output unit is configured to cause the training content to be displayed on the screen by outputting the training content.

6. The dementia determination system according to claim 4 or 5, wherein
the output unit is configured to cause the training content to be displayed on the screen by outputting the training content set for the dementia level determined by the determination unit.

7. The dementia determination system according to any one of claims 4 to 6, wherein
the acquisition unit is configured to newly acquire the long press ratio in a determination target period including a training period in which the training content is displayed, and
the determination unit is configured to newly determine the dementia level based on the long press ratio newly acquired by the acquisition unit.

8. A program for causing a computer to execute a dementia determination process, the dementia determination process comprising:
acquiring a long press ratio which is a ratio of a number of long presses in which a button of an operating device is pressed for a time longer than a reference time, to a number of presses of the button of the operating device used by a user to operate an electric device;
determining a dementia level of the user based on the long press ratio acquired by the acquiring; and
outputting dementia information indicating the dementia level determined by the determining, wherein
in the determining, the dementia level is more severe as the long press ratio is lower.

9. A dementia determination system, comprising:
an acquisition unit configured to acquire a duration of pressing a button of an operating device used by a user to operate an electrical device;
a determination unit configured to determine a dementia level of the user based on a distribution of the duration of pressing acquired; and
an output unit configured to output dementia information indicating the dementia level determined.

10. The dementia determination system according to claim 9, wherein
the determination unit is configured to determine the dementia level of the user by comparing the distribution in an intermediate section when the duration of pressing is divided into three continuous sections and the distribution in other two sections of the three continuous sections.

11. The dementia determination system according to claim 10, wherein
when A is an integral value of the distribution in a first section immediately before the intermediate section, B is an integral value of the distribution in the intermediate section, and C is an integral value of the distribution in a second section immediately after the intermediate section, the determination unit is configured to determine the dementia level of the user based on a feature value represented by (B/A) + (B / C).

12. The dementia determination system according to claim 11, wherein
when the feature value is greater than or equal to a threshold value, the determination unit is configured to determine the dementia level to be an abnormal level corresponding to mild cognitive impairment.

13. The dementia determination system according to any one of claims 10 to 12, wherein
the intermediate section is a section in which the duration of pressing is 1000 ms or more and 3000 ms or less.

14. The dementia determination system according to claim 9, wherein
the determination unit is configured to determine the dementia level of the user based on a temporal change in the distribution.

15. The dementia determination system according to any one of claims 9 to 14, wherein
the distribution is calculated by averaging a relative frequency distribution of the duration of pressing for one day over a plurality of days.

16. A program for causing a computer to execute a dementia determination process, the dementia determination process comprising:
acquiring a duration of pressing a button of an operating device used by a user to operate an electrical device;
determining a dementia level of the user based on a distribution of the duration of pressing acquired; and
outputting dementia information indicating the dementia level determined.
